(19) Europäisches Patentamt
European Patent Office
Office européen des brevets

(11) EP 3 735 585 B1

(12) **EUROPEAN PATENT SPECIFICATION**

(45) Date of publication and mention
of the grant of the patent:
**15.03.2023 Bulletin 2023/11**

(21) Application number: **18705317.8**

(22) Date of filing: **03.01.2018**

(51) International Patent Classification (IPC):
**G01N 29/07** *(2006.01)*     **A61B 8/08** *(2006.01)*
**A61B 8/00** *(2006.01)*     **B06B 1/06** *(2006.01)*
**G01N 29/26** *(2006.01)*     **G01S 15/89** *(2006.01)*

(52) Cooperative Patent Classification (CPC):
**G01N 29/07; A61B 8/0875; A61B 8/4494;**
**A61B 8/5223; B06B 1/0629; G01N 29/262;**
**G01S 7/52046; G01S 7/52049; G01S 15/8913;**
**G01S 15/8925; G01S 15/8927; G16H 50/30;**
G01N 2291/011; G01N 2291/02483;
G01N 2291/106

(86) International application number:
**PCT/EP2018/050114**

(87) International publication number:
**WO 2019/134742 (11.07.2019 Gazette 2019/28)**

(54) **SENSOR ARRANGEMENTS FOR AN ULTRASONIC PROBE**

SENSORANORDNUNGEN FÜR EINE ULTRASCHALLSONDE

AGENCEMENTS DE CAPTEURS POUR UNE SONDE À ULTRASONS

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(43) Date of publication of application:
**11.11.2020 Bulletin 2020/46**

(73) Proprietor: **Christian-Albrechts-Universität zu Kiel**
**24118 Kiel (DE)**

(72) Inventors:
• **GRÄSEL, Melanie**
**22305 Hamburg (DE)**
• **BARKMANN, Reinhard**
**24118 Kiel (DE)**
• **GLÜER, Claus-Christian**
**22587 Hamburg (DE)**

(74) Representative: **Hertin und Partner**
**Rechts- und Patentanwälte PartG mbB**
**Kurfürstendamm 54/55**
**10707 Berlin (DE)**

(56) References cited:
**US-A1- 2011 112 404     US-B1- 7 112 173**

## Description

The invention relates to sensor arrangements for an ultrasonic probe, wherein the sensor arrangement comprises emitters and receivers and wherein the emitters and receivers are arranged in rows and columns. In further aspects, the invention relates to a method for determining the difference in time of flight between two ultrasonic signals and to the use of the sensor arrangements.

## Prior Art:

Osteoporosis is a disease, which often leads to an increased risk of bone fracture. It is a disease, which becomes more common with age and particularly women are affected. The increased risk of bone fracture is due to an increased weakness of the bone, which may lead to fractures which occur with minor stress or spontaneously.

In particular, it has been found that osteoporosis is due to a reduced bone mass and/or an increased bone loss. In order to assess whether or not a patient suffers from osteoporosis, X-ray absorption or ultrasound measurements can be carried out. Dual energy X-ray absorptiometry measurements are useful in order to measure the areal bone mineral density (aBMD) and the osteoporotic fracture risk of a patient. However, this method depends strongly on the bone mass and bone size, but does not provide any information about structural parameters and/or bone material properties. Other X-ray methods, such as High Resolution peripheral Quantitative Computer Tomography (HR-pQCT), are prone to motion artefacts and limited to peripheral measurement sites, such as the distal radius or the distal tibia. In particular, the above-mentioned methods are not capable of segmenting structures in the image, which are smaller than 100 $\mu$m. A resolution in this range would, however, be required in order to differentiate healthy bone material from osteoporotic bone material. In an osteoporotic cortical bone material, the pore sizes vary between 7 and 400 $\mu$m, whereas in a healthy bone, the mean pore size is approximately 60 $\mu$m. Another disadvantage of X-ray methods is the radiation exposure of a patient which should be kept at a minimum.

With regard to ultrasound methods, the axial transmission technique is known, wherein the ultrasound velocity along the axis of long bones is measured. An example of a "long bone" are the radius or the tibia. It has been found that the squared velocity of an ultrasonic signal in a bone correlates with the effective elastic modulus in the linear propagation regime. Therefore, said ultrasound velocity can be used to estimate the elasticity of bone material regions of interest. In the context of the present invention, it is preferred to use the terms "velocity of an ultrasonic signal" and "speed of sound" synonymously. Preferably, the abbreviation SOS is used for the term "speed of sound".

United States Patent specifications US 7112173 B1 and US 2011/112404 A1 disclose array sensor arrangements for bone inspection, wherein each cell of the array can be individually accessed and each cell can be operable as a receiver, a transmitter, a transmitter/receiver or as non-active.

It is known that the bone structure consists of essentially tubular, cortical and spongy, trabecular bone material. The cortical bone material forms the outer shell of the bone and is usually a strong and dense structure made of woven fibers. This outer shell surrounds the sponge-like trabecular bone material. In the state of the art, in particular quantitative ultrasound measurements have been carried out with regard to the trabecular bone material in order to assess the osteoporotic fracture risk of a patient. However, it has been found that cortical bone properties, i.e. properties of the cortical bone material and structure, also contribute to the strength of a bone and influence the propagation speed of an ultrasonic signal through the cortical bone material. Examples of such fragility-relevant cortical bone properties are the porosity, mineralization or the thickness of the cortical region of the bone. However, it has for example not been possible so far to discriminate between porosity and mineralization in a bone with a single measurement.

It is therefore the object of the present invention to provide sensor arrangements for an ultrasonic probe and a method for determining the difference in time of flight between ultrasonic signals to overcome the shortcomings and disadvantages of the prior art. In particular, it is the object of the present invention to provide an ultrasonic device which is capable of measuring different fragility-relevant cortical bone properties, such as the cortical porosity and the cortical thickness, with a single measurement and to reduce the radiation exposure for the patient. A person skilled in the art would appreciate if a device could be provided, which is capable of carrying out *in vivo* measurements of a number of fragility-relevant cortical bone properties, such as cortical porosity and cortical thickness. A further object of the present invention is to provide sensor arrangements and a method for determining the difference in time of flight between ultrasonic signals, which is well suited for the use in clinical settings.

## Description of the invention:

The object underlying the present invention is solved by the features of the independent claims. Preferred embodiments of the invention can be found in the dependent claims. According to the invention, a sensor arrangement for an ultrasonic probe is provided, wherein the sensor arrangement comprises emitters and receivers and wherein the emitters and receivers are arranged in rows and columns. The sensor arrangement is characterized in that the sensors are arranged in rows of emitters and rows of receivers, wherein the columns are formed from upper emitters, upper receivers, lower receivers and lower emitters, wherein the sensor arrangement comprises electronics configured to excite and receive ultrasonic signals and to measure a velocity of an ultrasonic signal along a number of different signal paths between emitters and receivers, wherein the number of different signal paths comprises axial signal paths and tilted signal paths, wherein the tilted signal paths are tilted compared to the axial signal paths at a tilting +/- angle alpha, the tilting angle alpha is in a range of 30 to 45 °.

In the context of the present invention, this sensor arrangement is preferably referred to as "proposed sensor arrangement" or "first sensor arrangement".

For the proposed sensor arrangement, it is preferred that a central distance between the rows of sensors is in a range of 5 to 20 mm, preferably in a range of 10 to 15 mm and most preferably at 13 mm. In the context of the present invention, the term "central distance" preferably relates to the distance between the centers of the sensors. Preferably, the central distance between the columns of sensors is in a range of 5 to 15 mm, preferably in a range of 8 to 12 mm and most preferably at 10 mm. When using the axial transmission technique, with which e.g. the cortical compartment of a long bone can be measured, at least one emitter and one receiver are placed on one side of the bone. The emitter is configured to excite an ultrasonic wave, which propagates through the coupling medium. In the context of the present invention, it is preferred to refer to the ultrasonic wave as ultrasonic signal. Preferably, the coupling medium can be formed from soft tissue or water. It is preferred that the ultrasonic signal impinges on the specimen, e.g. a bone or a region of interest of a bone. The part of the ultrasonic signal, which impinges on the specimen under a critical angle, preferably travels along the surface of the specimen, wherein energy is preferably constantly emitted back into the coupling medium, e.g. the soft tissue surrounding the bone. This energy, which is emitted into the coupling medium, can be detected by a receiver or a receiving sensor.

In the context of the present invention, it is preferred that there is a minimum distance between the emitters and the receivers, which form the proposed sensor arrangement in order to receive the ultrasonic wave, which travels through the specimen, before the ultrasonic wave passes through the coupling medium. It is preferred that two ultrasound signals are detected. Preferably, the first ultrasound signal is related to a first receiver and the second ultrasound signal is related to a second receiver, wherein both ultrasound signals preferably travel through the bone. In the context of the present invention, the period of time, which is needed by the first ultrasonic signal and the second ultrasonic signal to travel through the bone and the soft tissue is preferably referred to as "time of flight" (TOF). The time difference between the detection of the first ultrasonic signal and the detection of the second ultrasonic signal is preferably referred to as "difference in time of flight" (ΔTOF). In the context of the present invention, it is preferred that the difference in time of flight is used in order to calculate the "speed of sound" (SOS), wherein the SOS preferably represents the velocity of the ultrasonic signal propagating through the bone. In the context of the present invention, it is preferred to use the point in time, where the signal amplitude reaches 10 % of the first positive amplitude, to calculate the TOF. In order to determine the SOS, it is preferred to use the TOF, which is preferably defined as the point in time, where the amplitude of the first arriving signal reaches 10% of its maximum. In the context of the present invention, it is preferred to use the terms "first positive elongation" and the term "first arriving signal" synonymously. Other methods for the determination of the TOF can also be used in the context of the present invention.

In one aspect, the invention relates to a method for determining the difference in time of flight between a first ultrasonic signal and a second ultrasonic signal. The difference in time of flight can preferably be used to assess the osteoporotic fracture risk of a material, in particular a bone material. Preferably, the method is configured to provide intermediate results, e.g. in order to evaluate the progress of the osteoporosis and/or the success of a treatment. In the context of the invention, it is preferred to further analyze the intermediate results preferably provided by the method, wherein the further analysis can e.g. be performed by means of information technology. It is preferred that the method can be carried out with the sensor arrangements as described in this document. In other words: the sensor arrangements described herein can be used in order to carry out the method described herein. The technical effects, definitions and advantages apply preferably analogously.

The proposed sensor arrangement is configured to measure a velocity of ultrasonic signals along a number of different signal paths. In particular, the sensor arrangement is configured to measure the velocity of at least one ultrasonic signal, preferably more ultrasonic signals. Preferably, the number of different signal paths includes a subset of axial and tilted signals paths. As an example, the measurements along an axial signal path may include a first ultrasonic signal, which is preferably emitted by the left upper emitter and received by the left upper receiver. Preferably, the second ultrasonic signal may be emitted by the left upper emitter and received by the left lower receiver. The same applies analogously for the central and the right column of the proposed sensor arrangement, preferably coinciding with the central and right axial signal path. For the tilted signal paths, it may e.g. be preferred that a first ultrasonic signal is emitted by the left upper emitter and received by the central upper receiver and that a second ultrasonic signal is emitted by the left upper emitter and received by the right lower receiver. In another example describing a tilted signal path, it may be preferred that a first ultrasonic signal is emitted by the right upper emitter and received by the central upper receiver and that a second ultrasonic signal is emitted by the right upper emitter and received by the left lower receiver. It is preferred that the first and second signals specified in these examples represent ultrasonic signals forming the positive direction of the preferably bidirectionally measured ultrasonic transmission, wherein the bidirectionality is explained further below in this document.

It is preferred that the difference in time of flight between the two ultrasonic signals is measured along a number of signal paths. Preferably, the number of signal paths can be 1 to 15, preferably 2 to 10 and more preferably 3 to 8, wherein all numbers between 1 to 15 may be preferred. It is most preferred that the differences in time of flight are measured along five different signal paths, wherein three signal paths preferably represent axial signal paths and the two remaining signal paths preferably represent tilted signal paths. Preferably, the measurements are carried out bidirectionally. In the context of the present invention, the term "bidirectional" preferably means that at least two measurements are carried out along each signal path, wherein the measurements are preferably referred to as "positive" and "negative" direction of the transmission of the ultrasound signal.

Preferably, the measurements performed along one signal path are carried out essentially in parallel. As an example, the measurements performed along an axial signal path may preferably only affect the receivers and emitters of said axial signal path. In other words, it is preferred that the measurements carried out along the axial signal paths are carried out along the same column of receivers and emitters of the proposed sensor arrangement. This means preferably that the ultrasonic signals may by emitted by the upper and lower emitter of a column of the sensor arrangement and that the signals may be received and/or detected by the upper and lower receiver of the same column of the sensor arrangement. In particular, the positive transmission measurements may start at the left upper emitter, i.e. the ultrasonic signals are emitted by the left upper emitter, and the negative transmission measurements start at the left lower emitter, i.e. the ultrasonic signals are emitted by the left lower emitter.

It is preferred that the tilted signal paths are tilted by a tilting angle *alpha* compared to the axial signal path. As an example, the positive tilted signal path may comprise positive measurements, where the measurements start at the left upper emitter, i.e. the first and second ultrasonic signal are preferably emitted by the left upper emitter, wherein the corresponding negative measurements start at the right lower emitter, i.e. the first and the second ultrasonic signal are preferably emitted by the right lower emitter. The first positive ultrasonic signal is preferably detected by the central upper receiver. The second positive ultrasonic signal is preferably detected by the right lower receiver. The first negative ultrasonic signal is preferably detected by the central lower receiver and the second negative ultrasonic signal is preferably detected by the left upper receiver. The course of preferred signal paths and preferred positions of the receivers and emitters are shown in more detail in the figures of this document.

Preferably, the columns preferably forming the proposed signal paths and/or the signal paths themselves comprise at least two receivers each, wherein the receivers are preferably configured to detect the ultrasonic signals emitted by the emitters.

The use of at least two receivers within a signal path is particularly advantageous in order to eliminate the influence of the thickness of the coupling medium, e.g. the soft tissue. It is preferred that at least two receivers are arranged in line with at least one emitter, preferably two emitters, wherein the sensors preferably form part of the proposed sensor arrangement. It is particularly preferred that the receivers and the emitters form a signal path, along which the propagating ultrasound signal, in particular its velocity, can be measured. The distance between the at least two receivers in one signal path is preferably referred to as distance s. The SOS, in particular the velocity of the ultrasonic signal propagating through the bone, can advantageously be calculated using the following equation:

$$SOS = s / \Delta TOF.$$

In the context of the present invention, it is particularly preferred to use a bidirectional axial transmission technique. The use of a bidirectional axial transmission technique is particularly advantageous in order to further improve the accuracy of the results of the measurement with regard to the often inconstant thickness of the coupling medium. In other words, using bidirectional axial transmission techniques advantageously helps to reduce the influence of the thickness of the coupling medium on the measurement and/or the quality of the measurement results. This is important as the thickness of the soft tissue, preferably forming the coupling medium, often varies along the site of the measurement. Studies show that a maximum soft tissue thickness is in a range of 0 to 4 mm. In the context of the analysis of the axial transmission technique, it is preferred that the ultrasound wave as a whole is analyzed and that the results are compared to theoretic models. For this analysis, it is preferred to plot the ultrasound signal, e.g. of a bone material, against the time.

It is particularly preferred to arrange a second emitter opposite of the first emitter within the columns preferably forming the proposed signal paths and/or within the signal paths, wherein the receivers and the emitters are preferably arranged in a line. Preferably, the SOS in preferably two directions can be measured making use of this preferred arrangement of sensors within a column of the proposed sensor arrangement. It is preferred that a first given direction can be referred to as "positive direction" or "+ direction", and that a second direction, which preferably represents the opposite direction of the preferred bidirectional axial transmission technique, can be referred to as "negative direction" or "- direction". The SOS values, which are obtained by the measurements in the positive and the negative direction, are preferably referred to as "positive SOS" (SOS$^+$) and "negative SOS" (SOS$^-$), respectively. Tests have shown that the harmonic mean of the two SOS values, in particular the harmonic mean of the positive SOS and the negative SOS, which can preferably be obtained by using the proposed sensor arrangement, is less susceptible to an inclination angel than each SOS value on its own. Preferably, the overall SOS can preferably be calculated using the following equation:

$$SOS = 2 / ( 1/SOS^+ + 1/SOS^- )$$

When developing the sensor arrangements according to the present invention, one particular challenge was that simulation studies indicated that the ultrasound velocity in tangential direction, i.e. perpendicular to the bone axis, is stronger influenced by deviations in porosity changes than the velocity in axial direction. The inventors therefore assumed that the squared ratio of the two velocities correlates with the porosity within the bone material of interest. This assumption led to the development of the proposed sensor arrangement, which is surprisingly capable of measuring the velocity, wherethrough the cortical porosity, e.g. of a human tibia, can advantageously be estimated *in vivo.* These advantageous effects are due to the advantageous arrangement of sensors of the ultrasonic probe. In the context of the present invention, the term "sensor" is preferably used for emitters and receivers, wherein the emitters are preferably configured to emit ultrasonic signals and wherein the receivers are preferably configured to receive and/or detect preferably modified ultrasonic signals. It is preferred that the modified ultrasonic signals are obtained after the propagation of the original ultrasonic signal through the bone region of interest. Preferably, the terms "ultrasound" and "ultrasonic" are used synonymously in the context of the present invention.

In the context of the present invention, it is most preferred that the method for determining the difference in time of flight between a first ultrasonic signal and a second ultrasonic signal, i.e. the method for assessing the osteoporotic fracture risk of a material, in particular a bone material, most preferably a bone material of a patient, comprises the following steps:

a) Providing a sensor arrangement, e.g. as described in this document,
b) Providing a first ultrasonic signal and a second ultrasonic signal,
c) Determining a time of flight (TOF) of the first and the second ultrasonic signal,
d) Determining a difference in time of flight (ΔTOF) between the first and the second ultrasonic signal,
e) Using the ΔTOF in order to determine a speed of sound (SOS).

It is particularly preferred that the cortical porosity of the examined bone material can be derived from the SOS as determined according the proposed method. Additionally, it is preferred that steps c) and d) can be repeated for a number of signal paths and/or in a positive and a negative direction. Preferably, the number of signal paths is five, including three axial and two tilted signals paths. Furthermore, it is preferred that the positive and the negative direction contribute to form a bidirectional transmission measurement. In the context of the present invention, it is preferred that the method for determining the difference in time of flight between a first ultrasonic signal and a second ultrasonic signal or the method for assessing the osteoporotic fracture risk of a material can be carried out with the proposed sensor arrangements, as described in this document. This preferably means that the proposed sensor arrangements, as described herein, can be used to carry out the above-mentioned method. It is particularly preferred that the invention additionally refers to the use of the sensor arrangements according to the present invention in order to determine a speed of sound of an ultrasound signal within a bone material.

It is preferred that the sensor arrangement comprises emitters and receivers, which are preferably arranged in rows and columns to form a grid of sensors. Preferably, the ultrasonic signals are emitted by the emitters of the sensor arrangement. Additionally, it is preferred that the arrival of the ultrasonic signals is detected by the receivers, wherethrough the TOF of the ultrasound signals can advantageously be determined. This can preferably be done by using a personal computer and/or suited electronic systems, as will be described later. The TOF of the ultrasound signals can be used in order to determine a difference in time of flight (ΔTOF) between the TOF of the ultrasound signals, wherein the ΔTOF can preferably be used in order to determine a speed of sound (SOS) of the ultrasound signals within a material of interest, e.g. a bone material.

Studies have shown that the curvature of the material of interest may influence the results of the SOS measurements, in particular the results of the tilted SOS measurements. In particular, the impact of the surface curvature of the bone material on the SOS measurements has been found to be direction-dependent. Therefore, it may be preferred that the analysis of the data obtained by the measurements may include a step of taking into account the curvature of the bone material. For this purpose, it is preferred to determine an estimated height $h_{est}$ of the axial signal paths above the surface of the bone. This is preferably done by taking into account the absolute TOF measured by one axial emitter-receiver combination, an SOS value measured within this axial signal path via bidirectional transmission, an assumed SOS of the coupling medium and the distance between the emitter and the receiver.

Preferably, the SOS$_{CM}$ of the coupling medium may be

$$SOS_{CM} = 1478 \text{ m/s.}$$

An estimated curvature elevation $e_{est}$ can preferably be derived as the difference between the estimated height $h_{est}$ calculated with a TOF of one of the outer axial signal paths and the estimated height $h_{est}$ calculated with the corresponding TOF of the central axial signal path. This determination can be repeated for all emitter-receiver-combinations and the calculated values for the curvature elevation can be averaged in order to obtain the estimated curvature elevation $e_{est}$. Preferably, the SOS values, which are obtained by measuring along the tilted signal paths, can be corrected with the curvature so determined. Improved results can be obtained, when the ultrasound probe is shifted and/or rotated with regard to the bone. The probe can also be tilted and/or lifted, wherein the results obtained can be averaged and/or compared.

In one preferred embodiment of the invention, it is preferred that the sensors are arranged in rows of emitters and rows of receivers. In the context of the present invention, it is particularly preferred that the sensor arrangement is formed from four rows of sensors, wherein a first row is formed from a row of upper emitters comprising three emitters, a second row is formed from a row of upper receivers comprising three receivers, a third row is formed from a row of lower receivers comprising three receivers and a fourth row is formed from a row of lower emitters comprising three emitters. In other words, the sensor arrangement comprises preferably six emitters and preferably six receivers, wherein three emitters form a row of upper emitters, where-in three receivers form a row of upper receivers, wherein three receivers form a row of lower receivers and wherein three emitters form a row of lower emitters. In a preferred embodiment of the invention, the rows of emitters and rows of receivers are arranged essentially equidistantly. In the context of the present invention, this preferably means that the distances between the sensor rows are essentially equal. It is particularly preferred that the sensor arrangement comprises three columns, which preferably comprise an essentially identical built-up. In a preferred embodiment of the invention, the columns are arranged essentially equidistantly to each other, i.e. they have essentially identical distances. Preferably, the proposed sensor arrangement is formed from a sensor pattern comprising twelve sensors, wherein preferably three emitters are arranged in an upper row of emitters, the receivers are arranged in two subsequent rows of preferably three receivers, which are preferably referred to as upper row and lower row of receivers, and a fourth row of preferably three lower emitters.

Preferably, each column of the proposed sensor arrangement comprises one upper emitter, one upper receiver, one lower receiver and one lower emitter. It is preferred that the columns are arranged essentially in parallel to each other, wherein the two outer columns enclose the middle or central column. It is preferred to refer to the two outer columns as left column and right column. Thus, each of the preferably twelve sensors of the sensor arrangement can individually be addressed with an appropriate denotation, such as left upper emitter, central upper receiver or right lower emitter. The denotations can easily be understood in connection with the figures representing the present invention. In connection with these figures, the person skilled in the art will easily understand terms, such as "right", "left", "upper" or "lower", or indications of direction, such as "below" or "above", so that these terms are not unclear in the context of the present invention. Additionally, the term "essentially" is not unclear for the person skilled in the art. The person skilled in the art knows that the terms "essentially parallel" or "essentially equidistant" are meant to mean that the corresponding elements are arranged in a parallel or an equidistant way, but that there may be small deviations in the range of some degrees ° or Millimeters (mm), for example due to the manufacture of the elements or the manufacture of the device as a whole.

Using the denotation described above, the first row of sensors preferably comprises the three upper emitters, i.e. a left upper emitter, a central upper emitter and a right upper emitter. The second row of sensors preferably comprises the three upper receivers, i.e. the left upper receiver, the central upper receiver and a right upper receiver. The third row of sensors preferably comprises the three lower receivers, i.e. the left lower receiver, the central lower receiver and a right lower receiver. The fourth row of sensors preferably comprises the three lower emitters, i.e. the left lower emitter, the central lower emitter and a right lower emitter.

These advantageous effects of the invention are particularly due to the measurement of the velocity of an ultrasonic signal along a number of different signal paths through the proposed sensor arrangement. In particular, the measurement along the preferably five signal paths enables for the estimation of the velocity in both the tangential and the axial direction, wherethrough the cortical porosity can advantageously be estimated *in vivo*. In a preferred embodiment of the invention, the number of signal paths comprises axial signal paths and tilted signal paths, wherein it is particularly preferred that the measurements comprise three axial signal paths and two tilted signal paths.

Preferably, the columns of the sensor arrangement coincide with the axial signal paths. In the context of the present invention, this means preferably that one axial signal path runs along the left sensor column, wherein a second axial signal path runs along the central column of sensors and the third axial signal path runs along the right sensor column. The axial signal paths are preferably referred to as left axial signal path, central axial signal path and right axial signal path, respectively. In other words, it is preferred that the sensor arrangement comprises a first column, a second column and a third column, wherein the first column preferably coincides with a left outer axial signal path, the second column preferably coincides with a middle axial signal path and wherein the third column preferably coincides with a right axial signal path.

As an example, the left axial signal path may comprise two ultrasound signals which are preferably emitted from the left upper emitter. The first ultrasound signal, which is emitted from the left upper emitter, is preferably detected by the left upper receiver, whereas the second ultrasound signal, which is emitted from the left upper emitter, is preferably detected by the left lower receiver. In the context of the present invention, it is preferred to refer to these two ultrasound signals as "positive" or "+" direction of the left axial signal path.

The left axial signal path may further comprise two ultrasound signals, which are preferably emitted from the left lower emitter. The first ultrasound signal, which is emitted from the left lower emitter, is preferably detected by the left lower receiver, whereas the second ultrasound signal, which is emitted from the left lower emitter, is preferably detected by the left upper receiver. In the context of the present invention, it is preferred to refer to these two ultrasound signals as "negative" or "-" direction of the left axial signal path. In the context of the present invention, it is preferred that the measurements carried out to form the positive direction and the negative direction of the transmission measurement, represent the bidirectionality of the transmission measurement.

Accordingly, the central axial signal path may comprise two ultrasound signals, which are preferably emitted from the central upper emitter. The first ultrasound signal, which is emitted from the central upper emitter, is preferably detected by the central upper receiver, whereas the second ultrasound signal, which is emitted from the central upper emitter, is preferably detected by the central lower receiver. These signals are preferably referred to as "positive" or "+" direction of the central axial signal path. The central axial signal path may further comprise two ultrasound signals which are preferably emitted from the central lower emitter. The first ultrasound signal, which is emitted from the central lower emitter, is preferably detected by the central lower receiver, whereas the second ultrasound signal, which is emitted from the central lower emitter, is preferably detected by the central upper receiver, where-in these latter signals are preferably referred to as "negative" or "-" direction of the central axial signal path.

Accordingly, the right axial signal path may comprise two ultrasound signals, which are preferably emitted from the right upper emitter. The first ultrasound signal, which is emitted from the right upper emitter, is preferably detected by the right upper receiver, whereas the second ultrasound signal, which is emitted from the right upper emitter, is preferably detected by the right lower receiver. These signals are preferably referred to as "positive" or "+" direction of the right axial signal path. The right axial signal path may further comprise two ultrasound signals, which are preferably emitted from the right lower emitter. The first ultrasound signal, which is emitted from the right lower emitter, is preferably detected by the right lower receiver, whereas the second ultrasound signal, which is emitted from the right lower emitter, is preferably detected by the right upper receiver, wherein these latter signals are preferably referred to as "negative" or "-" direction of the right axial signal path. It is preferred that the axial signal paths run essentially in parallel to each other.

Next to the axial signal paths, it is preferred that the number of signal paths additionally comprises two tilted signal paths. Preferably, the tilted signal paths are tilted compared to the axial signal paths at a +/- tilting angle alpha. The +/- tilting angle alpha can be determined as follows: A virtual straight line can be thought to run through the central sensor column. A tilted virtual straight line can for example be thought to run through the central upper receiver and the right upper emitter. At the central upper receiver, in particular on its right side seen from the virtual straight line, the two virtual straight lines enclose the tilting angle alpha. The orientation of this tilting angle alpha is preferably referred to as the "negative" tilted angle and the corresponding signal path is preferably referred to as "negative" tilted signal path.

The "positive" tilted signal paths can for example be obtained by virtually putting a virtual straight line through the central censor column and putting a tilted virtual straight line through, e.g., the central upper receiver and the left upper emitter. At the central upper receiver, in particular on its left side seen from the virtual straight line running through the central sensor column, the two virtual straight lines enclose the positive tilting angle alpha. The corresponding signal path is preferably referred to as "positive" tilted signal path.

In the context of the present invention, it is preferred that the distances of the rows and/or columns of the proposed sensor arrangement are arranged in way that the preferred +/- tilting angle alpha is arrived at. In particular, the emitters and receivers forming the proposed sensor arrangement are arranged so that the preferred +/- tilting angle alpha between the axial signal paths and the tilted signal paths is arrived at. In other words, it is preferred that the size of the tilting angle alpha can be used to determine the distances between the sensors of the proposed sensor arrangement. Preferably, the arrangement of the emitters and receivers depends from the choice of the size of tilting angle alpha.

In the context of the present invention, it is preferred that the negative tilted signal path comprises two ultrasound signals, which are preferably emitted from the right upper emitter. The first ultrasound signal, which is emitted from the right upper emitter, is preferably detected by the central upper receiver, whereas the second ultrasound signal, which is emitted from the right upper emitter, is preferably detected by the left lower receiver. These signals are preferably referred to as "positive" or "+" direction of the negative tilted signal path. The negative tilted signal path may further comprise two ultrasound signals which are preferably emitted from the left lower emitter. The first ultrasound signal, which is emitted from the left lower emitter, is preferably detected by the right upper receiver, whereas the second ultrasound signal, which is emitted from the left lower emitter, is preferably detected by the central lower receiver, wherein these latter signals are preferably referred to as "negative" or "-" direction of the negative tilted signal path.

Accordingly, it is preferred that the positive tilted signal path comprises two ultrasound signals, which are preferably emitted from the left upper emitter. The first ultrasound signal, which is emitted from the left upper emitter, is preferably detected by the central upper receiver, whereas the second ultrasound signal, which is emitted from the left upper emitter, is preferably detected by the right lower receiver. These signals are preferably referred to as "positive" or "+" direction of the positive tilted signal path. The positive tilted signal path may further comprise two ultrasound signals which are preferably emitted from the right lower emitter. The first ultrasound signal, which is emitted from the right lower emitter, is preferably detected by the central lower receiver, whereas the second ultrasound signal, which is emitted from the right lower emitter, is preferably detected by the left upper receiver, wherein these latter signals are preferably referred to as "negative" or "-" direction of the positive tilted signal path.

In the context of the present invention, it is preferred that the positive and negative direction measurements of the tilted signal paths are carried out essentially in parallel. Preferably, they are not carried out using identical emitters and receivers, as is preferably the case with regard to the axial signal paths. Preferably, the directions of the signal paths, which may be positive or negative, represent the bidirectional transmission pathways, which are used to measure the velocity of the ultrasonic signals, which are preferably emitted by the emitters and received and/or detected by the receivers of the proposed sensor arrangement.

It was challenging arriving at the proposed sensor arrangement, as it was found that a minimum transducer distance is required for axial ultrasonic measurements in order to receive the ultrasound wave propagating through the bone earlier than the ultrasound wave propagating through the soft tissue only. The challenge particularly arises from the limited width of the human tibia, which makes is difficult to measure a bidirectional transmission in tangential direction. This is particularly due to the fact that not in all bones the minimal required transducer separation can be achieved. In order to overcome this challenge, the inventors propose to make use of the cosine behaviour of the propagation velocity of an ultrasound signal. As bone material preferably represents a transverse isotropic medium, the velocity profile preferably follows a cosine curve with its maximum in axial direction and its minimum in tangential direction. In order to obtain said cosine dependency, the SOS within a cortical bone material can be measured under different measuring angles and the results for the SOS can be plotted against the angles. It is particularly preferred to use the human tibia for these measurements. These findings lead to the proposed design of the sensor arrangement, wherein the proposed grid of sensors is surprisingly capable of measuring ultrasound velocities in preferably five different signal paths, wherein three axial signal paths preferably run in parallel to each other and two signal paths are preferably tilted compared to the axial signal paths.

According to the invention, the tilting angle *alpha* is in a range of 30 to 45 °, preferably in a range of 35 to 40 °, most preferably at 37.5 °. It came as a surprise that it is possible to estimate the ultrasonic signal velocity in tangential direction, when measuring the velocities under different angles, as preferably represented by the number of different signal paths. Advantageously, the challenge created by the transverse isotropic nature of bone material with regard to the propagation of ultrasonic signals through a bone can surprisingly be overcome with the present invention. The preferred sensor arrangements according to the present invention surprisingly allow to obtain additional information about the bone material due to its transverse isotropic nature. Thus, the invention points in an opposite direction compared to the state of the art, where it is assumed that the transverse isotropic nature of the bone material represents an undesired source of errors.

In the context of the present invention, it is preferred to combine the results of the measurements for the axial velocity, which is preferably measured along the axis of the bone, with the results for the velocity in tangential direction in order to obtain an anisotropy index which advantageously correlates with the cortical porosity of a bone region of interest. It is preferred to obtain a calibration curve of this correlation on human tibia bone *ex vivo*. Surprisingly, tests have shown that an ultrasonic probe comprising the proposed sensor arrangement is less prone to additional error sources, which may e.g. arise from variable curvature of the surface of a bone.

The most preferred size of the tilting angle *alpha* of 37.5 ° has been derived by the inventors by making use of the limited width of some human bones. In particular, the signal paths, which are preferably determined by the arrangement of the sensors, are chosen so that the preferably five signal paths arrive at measuring the bone essentially at the same time. Preferably, the measurement can be carried out without the need of moving the probe. Tests have shown that some bones have a width of only 20 mm. In particular for these bones, the most preferred size of the tilting angle *alpha* of 37.5 ° advantageously enables an essentially simultaneous measurement of the preferably five signal paths. This limited width of some bones advantageously led the inventors to arrange the left column of sensors and the right columns of sensor in a center distance of preferably 20 mm.

The most preferred size of the tilting angle *alpha* of 37.5 ° was also chosen as a compromise between the damping of the ultrasonic signals and the thickness of the soft tissue. In particular, the most preferred size of the tilting angle *alpha* of 37.5 ° has shown to be surprisingly well suited to be used in combination with the bidirectional transmission technique, which is preferably used in the context of the present invention. Thus, the most preferred size of the tilting angle *alpha* of 37.5 ° is not chosen arbitrarily, but takes into account the circumstances and constraints of the SOS measurements to be carried out with the ultrasound probe. Other preferred angle sizes, which can advantageously be used for the SOS measurements, are 14.4 °, 21 ° or 60 °.

In another aspect of the invention, the sensor arrangement comprises additional rows of emitters and an additional row of receivers, whereby a modified sensor arrangement is preferably obtained. It is preferred that the present invention additionally relates to this modified sensor arrangement, which can also be used in connection with an ultrasonic probe. It is preferred that the modified sensor arrangement comprises two additional rows of emitters, wherein these two rows of emitters are arranged outermost within the modified sensor arrangement. In other words, a first additional row of emitters is arranged above the row of upper emitters of the first sensor arrangement and the second additional row of emitters is arranged below the row of lower emitters of the first sensor arrangement. In other words, it is preferred that the additional rows of emitters are arranged as a row of additional upper emitters above the row of upper emitters of the first sensor arrangement and as a row of additional lower emitters below the row of lower emitters of the first sensor arrangement. It is preferred that the additional rows of emitters comprise three emitters, wherein the position of the three emitters corresponds to the left, central and right position of the sensors in the sensor rows of the proposed sensor arrangement. It is preferred that the denotations describing the sensor arrangement apply to the modified sensor arrangement as well.

In the context of the present invention, it is preferred that the additional rows of emitters have a distance to the rows of emitters of the first sensor arrangement in a range of 3 to 15 mm, preferably 4 to 10 mm and most preferred at 6.5 mm. It is preferred to indicate distances between sensor as the distance between the centers of the sensors. In other words: the first row of additional emitters is preferably arranged 3 to 15 mm, preferably 4 to 10 mm and most preferably 6.5 mm above the row of upper emitters of the first sensor arrangement and the second row of additional emitters is preferably arranged 3 to 15 mm, preferably 4 to 10 mm and most preferably 6.5 mm below the row of lower emitters of the first sensor arrangement. These distances preferably represent center distances. If a center distance of preferably 6.5 mm is used between the outermost additional rows of emitters and the row of upper and lower emitters of the first sensor arrangement, the distance advantageously represents one half of the preferred center distance of 13 mm between the sensor rows of the first sensor arrangement. This arrangement facilitates the manufacture of the ultrasound probe and the precision of the measurements.

The modified sensor arrangement preferably comprises an additional row of receivers, wherein the additional row of receivers preferably comprises five receivers. The inventors have found that the SOS measurements can advantageously be improved and made more precise, if preferably one receiver is added to each of the preferably five signal paths. In particular, the presence of the additional receivers allows for measurements in cases, where the bone to be measured is surrounded by thick layers of soft tissue. It is preferred that the additional row of receivers is arranged between the existing rows of receivers of the first sensor arrangement. These preferably five receivers are preferably referred to as additional receivers. It is preferred that the two outer additional receivers are arranged in line with the left and the right column of the proposed sensor arrangement. These additional receivers are preferably referred to as left and right additional receiver, respectively. Preferably, the central of the preferably five additional receivers forming the additional row of receivers is in line with the central column of the proposed sensor arrangement. Two further additional receivers are preferably arranged on either side of the central receiver of the additional row of receivers. In particular, one additional receiver is arranged between the left and the central additional receiver and one additional receiver is arranged between the right and the central additional receiver. Preferably, these two further additional receivers are referred to as first and second further additional receiver, respectively. It is preferred that the two further additional receivers are arranged in the middle between the central additional receiver and the left and right additional receivers. In the context of the present invention, the term "being arranged in the middle between two objects A and B" preferably means that the distance to object A is essentially equal to the distance to object B. In other words: the object "in the middle" has preferably the same distance to object A and object B. In the context of the present invention, it is preferred that the center distance of the preferably two further additional receivers is 5 mm to the neighboring sensors, particularly the neighboring additional receivers.

The modified sensor arrangement advantageously enables the *in vivo* measurement of human bone material. In particular, the cortical porosity and the cortical thickness of a bone material to be analyzed can be estimated. Tests have shown that the modified sensor arrangement is particularly useful for cases, where the bone material is covered by thick layers of soft tissue. Studies of the inventors show that this applies to about 50 % of the probands, who took part in the study. Thus, the use of the modified sensor arrangement enables for the first time that *in vivo* measurements can be carried out for a significant share of persons, whose osteoporotic bone fracture risk shall be determined. Tests of the inventors also show that the precision of the SOS measurements can significantly be improved, when the modified sensor arrangement is used for the measurements. This is due to the additional sensors in the modified sensor arrangement, which particularly enable to detect the ultrasound wave travelling through bone material not being superimposed by a wave travelling through soft tissue. Due to the advantageous discrimination between bone material and soft tissue, the measurement using the ultrasound probe with the modified sensor arrangement can be carried out on living patients. In particular, the use of the modified sensor arrangement allows the measurement of ultrasound waves, which travel through a bone, even if overlying soft tissue is present. It is preferred that the modified sensor arrangement can be used to carry out the method for determining the difference in time of flight between a first ultrasonic signal and a second ultrasonic signal and/or the method for assessing the osteoporotic fracture risk of a bone material.

Advantageously, a modified evaluation algorithm facilitates the *in vivo* measurements on the living patient, particularly if thick layers of soft tissue are present. Tests have shown that soft tissue layers, which may be up to 8 mm thick, can advantageously be measured with the modified sensor arrangement. Advantageously, the modified algorithm is extended compared to the evaluation algorithm for the proposed sensor arrangement in order to account for circumstances, which may be found in clinical settings, such as a varying velocity of the ultrasonic signal propagating through the bone, positioning errors of the probe during the measurement, the presence of soft tissue and/or the bone geometry, such as the curvature of the bone.

When the ultrasound wave, in particular the ultrasonic signal, travels through the material to be analyzed, e.g. bone material, the ultrasonic signal may undergo a change. Changes may occur with regard to frequency and/or signal amplitude of the ultrasound signal. The person skilled in the art is familiar with the meaning of the terms "frequency" and "signal amplitude" in the context of ultrasound signals. The inventors have found that the frequency and/or signal amplitude advantageously improve the estimation of the cortical porosity and the cortical thickness of a material to be analyzed, otherwise preferably based on SOS-data. In particular, the modified sensor arrangement allows for the measurement of ultrasonic waves and/or signals within a bone material, even when overlying soft tissue is present. This is advantageously due to the incorporation of the parameters frequency and/or signal amplitude into the analysis. Additionally, the improvements achieved with the modified sensor arrangement are advantageously arrived at thanks to the additional sensors forming the modified sensor arrangement, i.e. the two additional rows of emitters and the additional row of preferably five receivers. It is noted that the specific arrangement of emitters and receivers forming the modified sensor arrangement can be understood in connection with the figures showing preferred embodiments of the invention.

It is preferred that the results of the axial SOS measurements can preferably be used in combination with the axial shift of the frequency of the ultrasound signals in order to determine the cortical porosity in the outer band of the cortex. In the context of the present invention, it is preferred that the outer band preferably relates to a surface up to a depth of 1 mm. Preferably, the term "axial SOS measurements" refers to the SOS measurements measured along the preferably three axial signal paths. Furthermore, it is preferred to use the results of the tilted SOS measurements in combination with the tilted shift of the frequency of the ultrasound signals in order to measure the cortical porosity in a deeper band, preferably starting at a depth of 1.5 mm going to the endost. Preferably, the term "tilted SOS measurements" refers to the SOS measurements measured along the preferably two tilted signal paths. Moreover, it is preferred to use the axial and/or tilted damping of the amplitude of the ultrasound signals in order to determine the cortical thickness of a bone.

In a preferred embodiment of the invention, it is preferred that the diameters of the emitters and/or receivers forming the modified sensor arrangement are smaller than the diameters of the emitters and/or receivers forming the proposed sensor arrangement. In particular, the modified sensor arrangement comprises emitters and receivers with reduced diameter. It is particularly preferred that the diameters of the receivers and emitters are in a range of 1 to 6 mm, preferably 1.3 to 3 mm, more preferably 1.5 to 2 mm and most preferably at 1.7 mm. The preferred diameter of 1.7 mm has shown to be particularly well suited to carry out the SOS measurements for patients with enhanced soft tissue thickness. Advantageously, the modified sensor arrangement allows for the measurement of additional signal paths and a larger variety of signal paths. In particular, the additional signal paths can be measured under different angles with regard to the angle of the bone to be measured.

Preferably, the axial signal paths, which are used in connection with the modified sensor arrangement, coincide with the columns of the modified sensor arrangement, wherein the columns of the modified sensor arrangement comprise four emitters and three receivers. It is preferred that the emitters of the modified sensor arrangement are arranged in two upper rows and two lower rows and the receivers of the modified sensor arrangement are arranged in three inner rows, wherein the central row of receivers is formed from additional receivers, preferably five additional receivers. For the modified sensor arrangement, it is preferred that the axial signal paths coincide with the columns of the sensor arrangement. In particular, ultrasound signals are preferably emitted by the emitters of the first and second additional row of emitters and by the emitters of the row of upper and lower emitters. It is preferred that the ultrasound signals are sent from each emitter of one axial signal path to all receivers of that same signal path. In the following example, this will be described as an example for the left column of sensors, which preferably coincides with the left axial signal path:

Within the left axial signal path, the ultrasound signals, which are emitted by the emitter of the first additional row of emitters, are preferably received and/or detected by the receivers of the upper and lower receivers and by the additional left receiver. Furthermore, ultrasound signals are emitted by the emitter of the upper row of emitters, wherein these ultrasound signals are preferably received and/or detected by the receivers of the upper and lower receivers and by the additional left receiver. Additionally, ultrasound signals are emitted by the emitter of the lower row of emitters, wherein these ultrasound signals are preferably received and/or detected by the receivers of the upper and lower receivers and by the additional left receiver, too. Moreover, ultrasound signals are emitted by the emitter of the second additional row of emitters, wherein these ultrasound signals are preferably received and/or detected by the receivers of the upper and lower receivers and by the additional left receiver. The exemplary description of the ultrasound signals preferably forming the left axial signal path can preferably be transferred to the central and right axial signal path.

For the modified sensor arrangement, it is preferred that the ultrasound signals preferably forming the tilted signal paths are emitted by the emitters of the row of upper and lower emitters preferably forming the first sensor arrangement, in particular by the left and right upper emitter and by the left and right lower emitter. It is preferred that the left upper emitter and the right lower emitter emit the ultrasound signals for the positive tilted signal path. Preferably, the ultrasound signals emitted by the left upper emitter are detected and/or received by the central upper receiver, the second further additional receiver and the right lower receiver. It is preferred that the ultrasound signals emitted by the left upper emitter, which are preferably detected and/or received by the central upper receiver, the second further additional receiver and the right lower receiver, form the positive direction of the positive tilted signal path. The tilted signal path may be tilted with a most preferred tilting angle *alpha* of 37.5 °.

Additionally, the ultrasound signals emitted by the right lower emitter are detected and/or received by central lower receiver, the first further additional receiver and the left upper receiver. It is preferred that these ultrasound signals form the negative direction of the positive tilted signal path. The measurements along the tilted signal paths are preferably referred to as "quasi-bidirectional", as the two measurement directions, i.e. the positive and the negative direction of the tilted signal paths, are not identical. Preferably, the positive and the negative direction of the tilted signal paths run essentially in parallel.

With regard to the negative tilted signal path, it is preferred that the ultrasound signals of the positive direction are emitted by the right upper emitter. Its ultrasound signals are preferably detected and/or received by the central upper receiver, the first further additional receiver and the left lower receiver. It is preferred that the ultrasound signals of the negative direction are emitted by the left lower emitter, wherein its ultrasound signals are preferably detected and/or received by central lower receiver, the second further additional receiver and the right upper receiver. It is preferred that negative tilted signal path may be tilted with a most preferred tilting angle *alpha* of 37.5 ° compared to the axial signal paths. Preferably, the size of the tilting angle is determined by the arrangement of the sensors forming a particular signal path. In the context of the present invention, this preferably means that the distances between the sensors forming a particular signal path, particularly the tilted signal paths, are arranged so, that a desired preferred tilting angle is arrived at.

It is preferred that the ultrasonic probe comprising the proposed sensor arrangement or the modified sensor arrangement is part of a system further comprising a personal computer (PC) and electronics specifically configured for the analysis of the data obtained with the proposed sensor arrangements. Preferably, the system represents one further aspect of the present invention. In the context of the present invention, it is preferred that the electronics are configured to amplify the receiving signals and/or to digitalize the signals at a resolution of 14 bit and/or with a frequency of preferably 40 MHz. Preferably, the electronics itself does not comprise any filters.

The proposed sensor arrangement or the modified sensor arrangement are advantageously configured to measure the propagation of ultrasound signals along different signal paths. In the context of the present invention, it is preferred that the system comprises transducers which are preferably configured to transduce electrical power into mechanical pressure and *vice versa.* In the context of the present invention, it is preferred that the transducers are configured to convert mechanical energy of the ultrasonic signals into electric energy, wherein the electric energy can be measured.

In the context of the present invention, it is particularly preferred to refer to the emitters and receivers of the proposed sensor arrangements as "transducers". It is preferred that the transducers have an essentially circular shape, wherein the essentially circular shape is preferably characterized by a diameter in a range of 1 to 10 mm, preferably 3 to 7 mm, most preferably of 5 mm. The preferred diameter sizes mentioned herein are particularly preferred for the first sensor arrangement. For the modified sensor arrangement, preferred diameter sizes may preferably be in range of 1 to 6 mm, preferably 1.3 to 3 mm, more preferably 1.5 to 2 mm and most preferably at 1.7 mm. For some applications, it may be preferred that the sensors have the shape of an octagon or other shapes.

Preferably, the center distance between the first row of additional emitters and the row of upper emitters is in a range of 3 to 15 mm, preferably 4 to 10 mm and most preferred at 6.5 mm. It is preferred that the center distance between the second row of additional emitters and the row of lower emitters is also in a range of 3 to 15 mm, preferably 4 to 10 mm and most preferred at 6.5 mm. Additionally, it is preferred that the distance between the row of additional receivers and the row of upper and lower receivers is in a range of 3 to 15 mm, preferably 4 to 10 mm and most preferred at 6.5 mm. A center distance of 6.5 mm is most preferred in the context of the present invention. It is preferred that the central distance between the columns of sensors is in a range of 5 to 15 mm, preferably in a range of 8 to 12 mm and most preferably at 10 mm. Preferably, the transducers have a center frequency of 1 MegaHertz (MHz), wherein a 3 dB bandwidth preferably corresponds to 4 %. It is preferred that the receivers are arranged at a central position of the sensor arrangement and/or in the middle of the ultrasonic probe. Preferably, the emitters are arranged on either side of the receivers, in other words in rows preferably arranged above and below the rows of receivers. It is preferred that an emitter section, preferably formed from the emitters or a group of emitters, is separated from a receiver section, preferably formed from the receivers or a group of receivers, by ditches, wherein the ditches are preferably configured to prevent ultrasound propagation through the ultrasonic probe itself.

It is preferred that the system further comprises transducer pitches, wherein the transducer pitch in axial direction is in a range of 5 to 30 mm, preferably 10 to 18 mm and most preferably at 13 mm. Tests have shown that a transducer pitch in axial direction of 13 mm is particularly suited to take into account the correlation between the transducer distance and the thickness of the coupling medium, e.g. the soft tissue. In the tangential direction, a transducer pitch may be in a range of 1 to 50 mm, preferably 5 to 30 mm and most preferably at 10 mm. The value of 10 mm has shown to be particularly well suited for the tangential transducer pitch because of the limited width of the object to be measured, wherein the object will usually be a human bone, for example a tibia or a radius. Minimum widths of the object to be measured are found to be in a range of 20 mm. In the context of the present invention, it is preferred that these values result in a pitch in a range of 10 to 25 mm, preferably 15 to 18 mm and most preferably at 16.4 mm in a 37.5 ° direction, i.e. when the tilted signal paths are tilted at a tilting angle of *alpha* = 37.5 ° compared to the axial signal paths.

It is preferred that the electronics of the system comprise six pulsers, wherein it is particularly preferred that each emitter is allocated one pulser. The signals can preferably be pre-amplified, amplified and/or multiplexed on the side of the receivers. This built-up is particularly preferred for the first sensor arrangement. For the modified sensor arrangement, it can be preferred to use one pulser. Preferably, the excitation pulse is distributed by a multiplexer and in that way sent to the emitters. Preferably, one pre-amplifier is present for each receiver on the side of the receivers. Furthermore, it is preferred that the signals are multiplexed essentially directly after being pre-amplified. It may be preferred to amplify the signals subsequently, preferably after the pre-amplifying and the multiplexing. Tests have shown that these built-ups and/or amplifying methods are particularly well suited to improve the usability of the ultrasound probe comprising the proposed sensor arrangements.

Preferably, the system further comprises six signal acquiring paths, wherein a signal acquiring path comprises a pre-amplifier and an amplifier. It is preferred that the gain of the amplifier is designed variable. It is preferred that the pre-amplifier is operated at 26 dB. Preferably, the amplified signals can be multiplexed, digitized to 14 bit at a clock frequency of 40 MHz and/or transferred to the PC, which is preferably part of the system. In the context of the present invention, it is preferred that the emitters of the proposed sensor arrangement are excited once at a time with a +/- 100 V, 1 MHz square signal for one period. Preferably, 10 signals can be stored for each emitter-receiver-combination, wherein the average of these signals can be calculated.

Preferably, the ultrasound probe comprising the proposed sensor arrangement or the modified sensor arrangement is configured to measure three bidirectional signals paths and two quasi bidirectional signal paths. It is preferred that the bidirectional signals paths can also be referred to as axial signal paths and that the quasi bidirectional signal paths can also be referred to as tilted signal paths. In the context of the present invention, it is preferred that the axial signal paths are in alignment with the axis of the bone which is to be measured. Preferably, the bone to be measured is a long bone, such as a human tibia or a human radius. In other words, the bidirectional signal paths are preferably in alignment with the long bone axis. The tilted signal paths are preferably referred to as "quasi" bidirectional signal paths, as they are configured to measure along ultrasound signal paths which are preferably essentially parallel, but not identical. In the context of the present invention, it is most preferred that the point in time, where the signal amplitude reaches 10 % of the first positive amplitude, is used to calculate the TOF of the ultrasonic signals. Advantageously, the SOS can be derived by way of calculation using the TOF of two ultrasound signals, in particular their difference in time of flight ($\Delta$TOF). It is preferred that the ultrasound probe comprising the sensor arrangement can be calibrated on acrylic glass and polycarbonate in order to determine the distances between the emitters and receivers.

In a further aspect, the invention relates to a method for determining a velocity of an ultrasonic signal, wherein the velocity of an ultrasonic signal is calculated from a time of flight of the ultrasonic signal, wherein the time of flight is calculated from a point in time when an amplitude of the ultrasonic signal reaches 10 % of the first positive amplitude. In order to obtain a SOS profile, the measurements can include a full rotation on the surface of the bone to be measured, wherein the measurement can be performed in steps of e.g. 15 °. As an example, three repositioning measurements can be carried out for each position, wherein each repositioning measurement may comprise 10 single measurements, wherethrough e.g. 100 averaged signals for each emitter-receiver-combination are obtained. Advantageously, the elastic modulus E can be obtained using the relationship

$$SOS = \sqrt{E/\rho} \, ,$$

wherein $\rho$ is the density of the measured material in the unit (kg/m$^3$). It is particularly preferred that the cortical porosity of the examined bone material can be derived from the SOS as determined according to the proposed method. The inventors assumed that a parameter, which is comparable to the anisotropy of the elastic coefficients, can be obtained by determining the squared ratio of the SOS values in the axial and in the tilted signal paths, preferably measured when the axial signal paths are in alignment with the pores within the bone material. Advantageously, an anisotropy index (AI) can be calculated according to the following equation:

$$AI = (SOS_{0°} / SOS_{37.5°})^2.$$

In this equation, the term "$SOS_{0°}$" preferably refers to the mean of the preferably three SOS values measured with the axial signal paths, whereas the term "$SOS_{37.5°}$" preferably refers to the mean of the preferably two SOS values measured with the tilted signal paths in the positive and negative 37.5° direction. It is preferred to determine the cortical porosity by means of a linear regression of actual porosity and the AI values. In the context of the present invention, it is most preferred that the methods described in this document can be carried out with the first sensor arrangement or with the modified sensor arrangement.

The invention is described by the following figures showing preferred embodiment of the invention.

Fig. 1     Preferred embodiment of the first sensor arrangement

Fig. 2     Preferred embodiment of the first sensor arrangement, in particular the course of the signal paths

Fig. 3     Preferred embodiment of the first sensor arrangement, in particular several potential positions of the tilting angle

Fig. 4     Preferred embodiment of the modified sensor arrangement

Fig. 5     Preferred embodiment of the modified sensor arrangement, in particular the course of the signal paths

Fig. 6     Preferred embodiment of the modified sensor arrangement, in particular the course of the signal paths

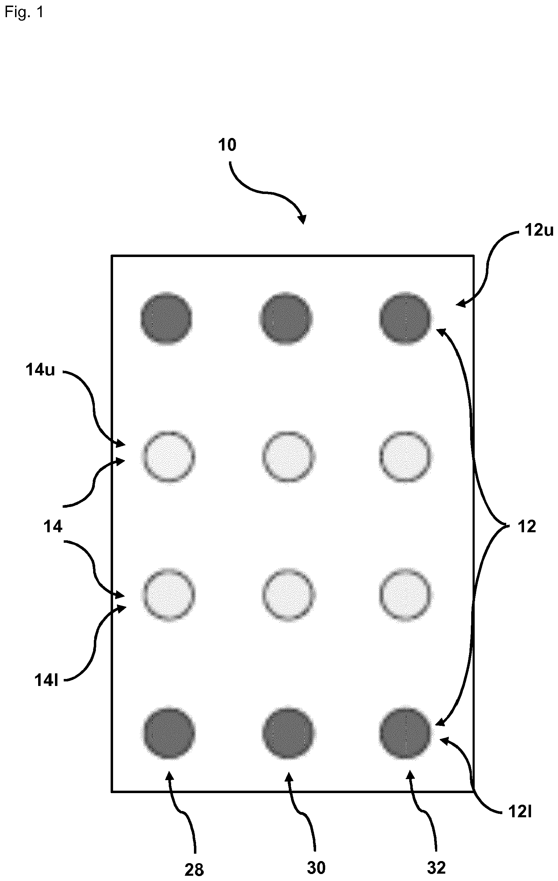

Figure 1 shows a preferred embodiment of the first sensor arrangement (10). In particular, figure 1 shows the rows of emitters (12) and receivers (14) forming a grid of sensors (12, 14), which represents an example of the first sensor arrangement (10) of the present invention. It is preferred that the emitters (12) are represented by dark circles and that the receivers (14) are represented by white circles. The preferred embodiment of the invention, which is shown in figure 1, comprises a row of upper emitters (12u), a row of upper receivers (14u), a row of lower receivers (141) and a row of lower emitters (121). In the context of the present invention, it is preferred that the term "12u" refers both to the row of upper emitters and to the single emitters of that row. This preferably applies *mutatis mutandis* to the upper receivers (14u), the lower receivers (141) and the lower emitters (121). Preferably, the emitters (12) and receivers (14) are referred to as sensors (12, 14) in the context of the present invention. In this example, it is also preferred that the distance between the rows is 13 mm. A preferred diameter of the sensors (12, 14) is 5 mm, but it may also be preferred that the sensors (12, 14) have alternative diameters, such as 1,7 mm. The preferred embodiment of the invention shown in figure 1 comprises three sensors (12 or 14) per row and three columns (28, 30, 32) of sensors (12, 14). The first column of the first sensor arrangement (10) is preferably referred to as left column (28) sensors (12, 14), the second column of the first sensor arrangement (10) is preferably referred to as central column (30) of sensors (12, 14) and the third column of the first sensor arrangement (10) is preferably referred to as left column (32) of sensors (12, 14). Preferably, the distance between the columns (28, 30, 32) is 10 mm in the preferred embodiment of the invention shown in figure 1.

It is preferred that the columns (28, 30, 32) coincide with ultrasonic signal paths (16), in particular the axial signal paths (16a), which are preferably used to carry out speed of sound (SOS) measurements within a bone material. It is preferred that the columns (28, 30, 32) of the first sensor arrangement (10) comprise one upper emitter (12u), one upper receiver (14u), one lower receiver (141) and one lower emitter (121). Preferably, the emitters (12u and 121) of a column (28, 30, 32) emit ultrasound signals and the ultrasound signals of the emitters (12u and 121) are received and/or detected by any of the receivers (14u and 141) of that column (28, 30, 32). As an example, the upper emitter (12u) of the right column (32) emits two ultrasound signals, which are received and/or detected by the upper receiver (14u) and the lower receiver (14l) of the right column (32).

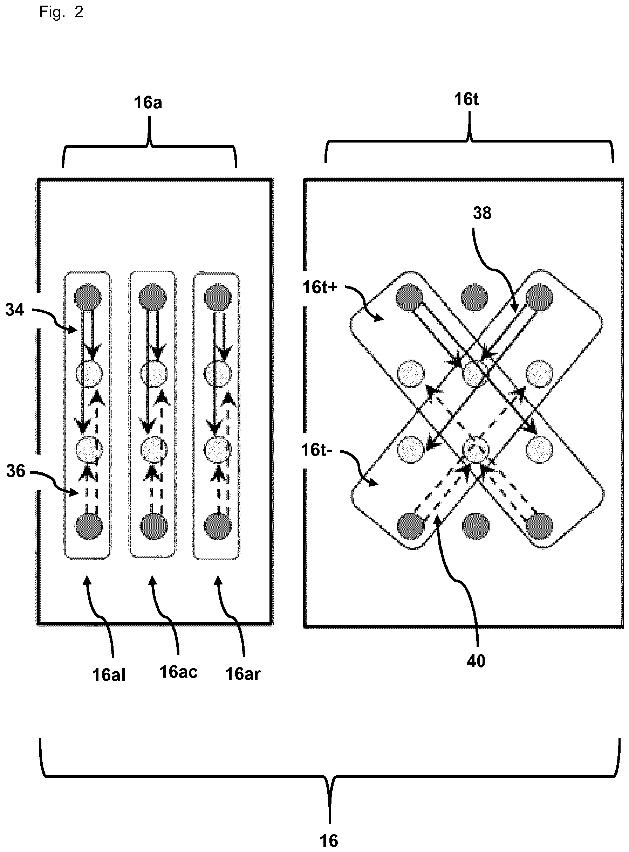

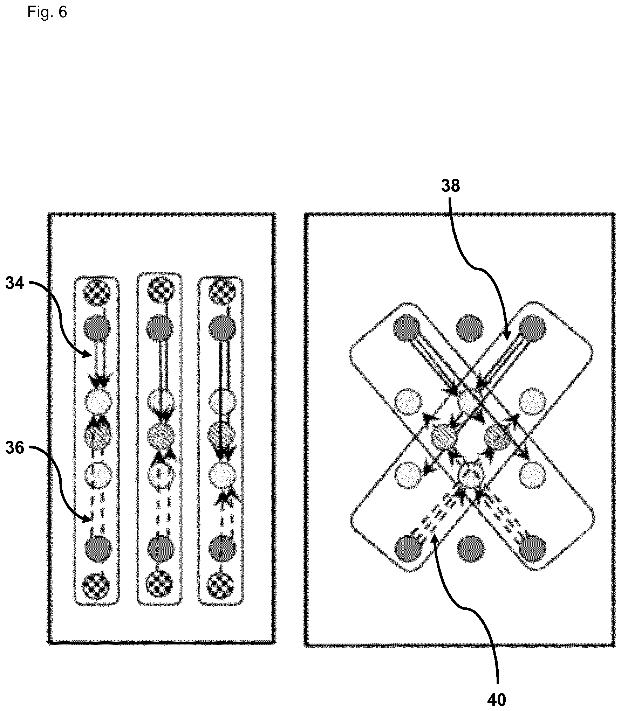

As can be seen from figure 2, the part of the right axial signal path (16ar) comprising the ultrasound signals emitted by the upper emitter (12u) of the right column (32) is preferably referred to as the positive direction of bidirectional transmission measurement (34), wherein this allocation can be transferred to the left column (28) and the central column (30) of the sensor arrangement (10). The positive directions (34, 38) of the signal paths (16, 26) are indicated with solid lines in the figures 2 and 6. The negative directions (36, 40) of the signal paths (16, 26) are indicated with dashed lines in the figures 2 and 6. In the above-mentioned example of the right column (32), the negative direction of the axial bidirectional transmission measurement (36) is formed from the ultrasound signals emitted by the right lower emitter (12l), which are preferably received and/or detected by the upper receiver (14u) and the lower receiver (14l) of the right column (32).

The sensor arrangement (10) according to the present invention is configured to measure a velocity of ultrasonic signals within a bone material along signal paths (16). It is preferred that the signal paths (16) comprise axial signal paths (16a) and tilted signal paths (16t), wherein the tilted signal paths (16t) are tilted compared to the axial signal paths (16a) at a tilting +/- angle *alpha* (18). Figure 2 shows preferred courses of the signal paths (16) for the first sensor arrangement (10). Preferably, the preferably three axial signal paths (16a) can be referred to as left axial signal path (16al), central axial signal path (16ac) and right axial signal path (16ar). It is preferred that the left axial signal path (16al) coincides with the left column (28) of the first sensor arrangement (10), that the central axial signal path (16ac) coincides with the central column (30) of the first sensor arrangement (10) and that the right axial signal path (16ar) coincides with the right column (32) of the first sensor arrangement (10).

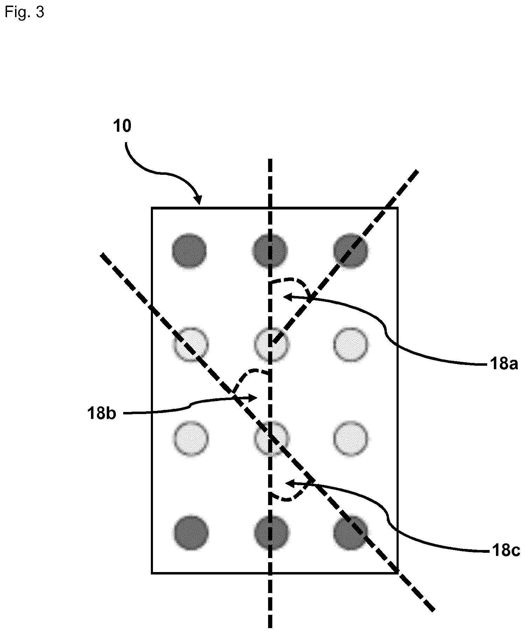

Figure 3 shows several potential positions of the tilting angle *alpha* (18). The tilting angle *alpha* (18) is indicated with a dashed bow between two dashed legs. It is preferred that the size of the tilting angle *alpha* (18) is determined by the positions and/or the arrangement of the sensors (12, 14) forming the sensor arrangements (10) proposed in this document. In particular, figure 3 shows examples of possible positions (18a, b, c), where the tilting angle *alpha* (18) can potentially be found within the proposed sensor arrangement (10). The virtual lines enclosing the tilting angle *alpha* (18) are indicated by dashed lines. A first central virtual line runs along and/or essentially in parallel with the central column (30), which preferably coincides with the central axial signal path (16ac). In particular, this first central virtual line runs through the central upper emitter (12u), the central upper receiver (14u), the central lower receiver (14l) and the central lower emitter (12l). A tilted virtual line starts at the central upper receiver (14u) and runs through the right upper emitter (12u), i.e. the upper emitter (12u) of the right column (32) of the first sensor arrangement (10). In the context of the present invention, it is preferred that three sensors (12, 14) of the sensor arrangement (10) enclose the tilting angle *alpha* (18).

The size of the tilting angle *alpha* (18) indicates how strong the tilted signal paths (16t) are tilted compared, for example, to the axial signal paths (16a). The tilting can preferably take place in a positive direction and in a negative direction. The preferably two tilted signal paths (16t) are preferably referred to as positive tilted signal path (16t+) and negative tilted signal path (16t-), depending on the direction of the tilting, e.g. compared to the axial columns (28, 30, 32) of the sensor arrangement (10). Preferably, the signal path, which is preferably formed from the ultrasound signals emitted by the left upper emitter (12u) and the right lower emitter (12l), is preferably referred to as positive tilted signal path (16t+). It is preferred that the signal path, which is preferably formed from the ultrasound signals emitted by the right upper emitter (12u) and the left lower emitter (12l), is preferably referred to as negative tilted signal path (16t-). It is most preferred that the tilting angle *alpha* (18) is 37.5 °, but it may also be preferred for other applications that tilting angle (18) of 14.4 °, 21 ° and/or 60 ° are used.

In figure 3, three possible positions (18a, b, c) of the tilting angle *alpha* (18) are indicated as examples. The first example (18a) of the position of the tilting angle *alpha* (18) is preferably determined by the central upper emitter (12u), the central upper receiver (14u) and the right upper emitter (12u). It is preferred that these three sensors (12u, 14u) enclose the tilting angle *alpha* (18) and determine one possible position within the first sensor arrangement (10). The second example (18b) of the position of the tilting angle *alpha* (18) is preferably determined by the central upper receiver (14u), the central lower receiver (14l) and the left upper receiver (14u). The third example (18c) of the position of the tilting angle *alpha* (18) is preferably determined by the central lower receiver (14l), the central lower emitter (12l) and the right lower emitter (12l). The person skilled in the art understands that 18b and 18c are vertically opposed angles, which are equal in size.

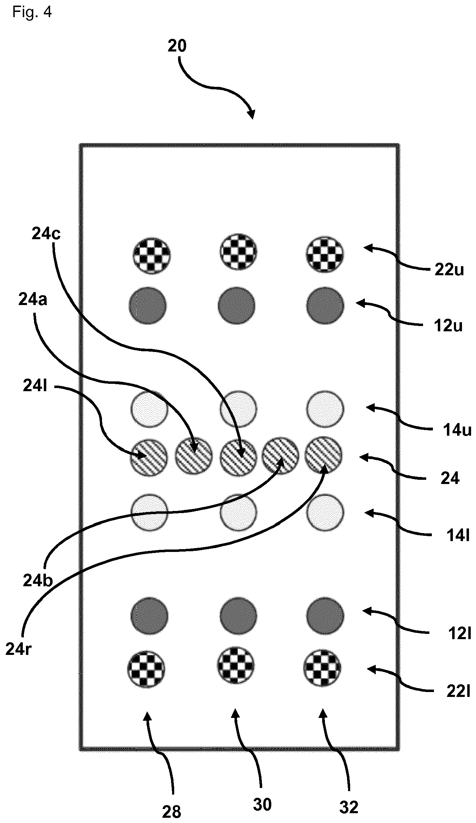

Figure 4 shows a preferred embodiment of the modified sensor arrangement (20). Preferably, the modified sensor arrangement (20) can be regarded as the first sensor arrangement (10), as far as the positions of certain sensors (12, 14) are concerned, plus additional rows of emitters (22) and one additional row of receivers (24). In other words, the modified sensor arrangement (20) preferably comprises the row of upper emitters (12u), the row of upper receivers (14u), the row of lower receivers (14l) and the row of lower emitters (12l), as described for the first sensor arrangement (10). These rows of sensors (12, 14) are preferably arranged as described for the first sensor arrangement (10), including the presence of a tilting angle *alpha* (18), which preferably indicates how strong the tilted signal paths (26t) are tilted compared to the axial signal paths (26a).

Additionally, the modified sensor arrangement (20) comprises additional upper emitters forming an uppermost row of emitters (22u). This uppermost row of emitters (22u) can preferably also be referred to as first row of additional emitters (22u). It is preferred that the first row of additional emitters (22u) is arranged above the row of upper emitters (12u) of the first sensor arrangement (10). Preferably, the distance between the centers of the additional emitters (22u) and the upper emitters (12u) is 6.5 mm. Furthermore, the modified sensor arrangement (20) comprises an additional row of lower emitters (22l) forming a lowest row of emitters (22l). It is preferred that this lowest row of emitters (22l) is referred to as second row of additional emitters (22l). The additional emitters (22u, 22l) of the additional rows of emitters (22u, 22l) are indicated with a chessboard patter in figures 4 to 6.

The modified sensor arrangement (20) further comprises an additional row of receivers (24). In figures 4 to 6, the additional receivers (24) are indicated with hatched circles. It is preferred that the additional row of receivers (24) is arranged between the row of upper receivers (14u) and the row of lower receivers (14l). It is particularly preferred that the additional row of receivers (24) is arranged in the middle between the row of upper receivers (14u) and the row of lower receivers (14l). In the context of the present invention, this preferably means that the additional row of receivers (24) has the same distance to the row of upper receivers (14u) and to the row of lower receivers (14l). Preferably, this distance is 6.5 mm, which preferably represents one half of the center distance of the rows of upper emitters (12u), upper receivers (14u), lower receivers (14l) and lower emitters (12l) of the first sensor arrangement (10), which is preferably 13 mm. It is most preferred that the additional row of receivers (24) comprises five receivers, wherein the left additional receiver (24l) can preferably be regarded as part of the left column (28) of the sensor arrangement (20), i.e. of the left axial signal path (26al). Preferably, the central additional receiver (24c) can preferably be regarded as part of the central column (30) of the sensor arrangement (20), i.e. of the central axial signal path (26ac). It is preferred that the right additional receiver (24r) can preferably be regarded as part of the right column (32) of the sensor arrangement (20), i.e. of the right axial signal path (26ar).

It is preferred that the additional row of receivers (24) comprises two further additional receivers (24a, 24b). Preferably, the first further additional receiver (24a) is arranged between the left additional receiver (24l) and the central additional receiver (24c). It is particularly preferred that the first further additional receiver (24a) is arranged in the middle between the left additional receiver (24l) and the central additional receiver (24c), i.e. it is preferred that the distance between the first further additional receiver (24a) and the left additional receiver (24l) is essentially the same as the distance between the first further additional receiver (24a) and the central additional receiver (24c). Preferably, the center distance between the two further additional receivers (24a, 24b) and their neighboring receivers is 5 mm. Preferably, the second further additional receiver (24b) is arranged between the right additional receiver (24r) and the central additional receiver (24c), wherein the second additional receiver (24b) is arranged in the middle between the right additional receiver (24r) and the central additional receiver (24c), i.e. it is preferred that the distance between the second further additional receiver (24b) and the right additional receiver (24r) is essentially the same as the distance between the second further additional receiver (24b) and the central additional receiver (24c). It is preferred that each additional receiver (24) is part of one of the preferably five signal paths (26), which the modified sensor arrangement (20) is preferably capable of measuring along. It is particularly preferred that the modified sensor arrangement (20) is configured to measure the speed of sound (SOS) in different directions within a bone material, so that an osteoporotic bone fracture risk can be determined from these intermediate results.

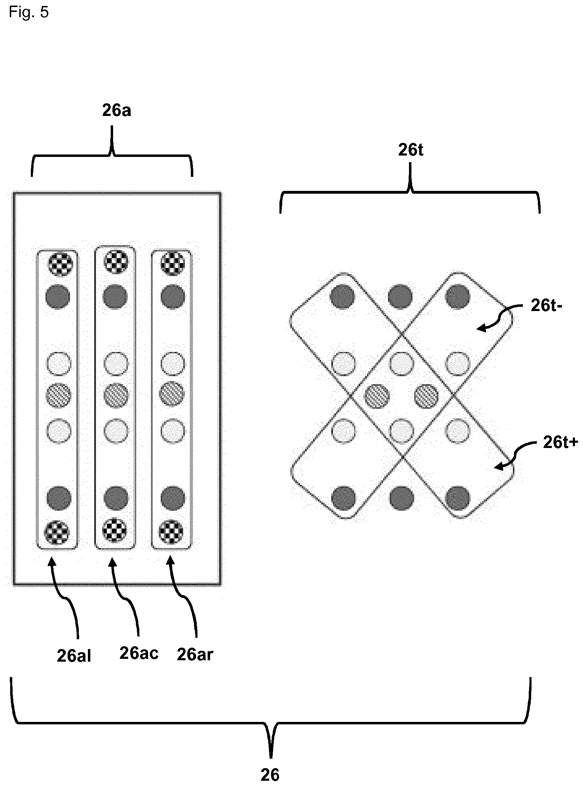

Exemplary courses of the signal paths (26) of the modified sensor arrangement (20) are shown in figures 5 and 6. Preferably, the modified sensor arrangement (20) is configured to measure the speed of sound (SOS) along five ultrasound signal paths (26), wherein there may be three axial signal paths (26a) and two tilted signal paths (26t) in the context of the modified sensor arrangement (20). The axial signal paths (26a) preferably coincide with the columns (28, 30, 32) of the sensor arrangement (20). It is particularly preferred that the left column (28) coincides with the left axial signal path (26al) of the modified sensor arrangement (20). Furthermore, it is preferred that the central column (30) coincides with the central axial signal path (26ac) of the modified sensor arrangement (20) and that the right column (32) coincides with the right axial signal path (26ar) of the modified sensor arrangement (20). Preferably, the columns (28, 30, 32) and axial signal paths (26a) of the modified sensor arrangement (20) comprise seven sensors, specifically a first or upper additional emitter (22u), an upper emitter (12u), an upper receiver (14u), an additional receiver (24), a lower receiver (14l), a lower emitter (12l) and a second or lower additional emitter (22l).

Preferably, the modified sensor arrangement (20) is also configured to measure the speed of sound (SOS) within a bone along two tilted signal paths (26t). These tilted signal paths (26t) are tilted by a tilting angle alpha (18) compared to the axial signal paths (26a). The most preferred size of the tilting angle alpha (18) is 37.5 °, but alternative sizes, such as 14.4 °, 21 ° or 60 °, are also conceivable. The tilted signal paths (26t) can be tilted in a positive direction or in a negative direction. It is preferred to refer to the tilted signal path (26t), which is preferably tilted in a positive angel direction, as positive tilted signal path (26t+) and to refer to the tilted signal path (26t), which is preferably tilted in a negative angel direction, as negative tilted signal path (26t-). It is preferred that the positive tilted signal path (26t+) comprises the left upper emitter (12u), the left upper receiver (14u), the central upper receiver (14u), the two further additional receivers (24a, 24b), the central lower receiver (14l), the right lower receiver (14l) and the right lower emitter (12l). Preferably, the negative tilted signal path (26t-) comprises the left lower emitter (12l), the left lower receiver (12l), the central upper receiver (14u), the two further additional receivers (24a, 24b), the central lower receiver (14l), the right upper receiver (14u) and the right upper emitter (12).

The SOS measurements carried out with the proposed sensor arrangements (10, 20) preferably make use of the bidirectional transmission technique. In the context of the bidirectional transmission technique, ultrasound signals are preferably emitted in two opposing directions within one signal path. Preferably, the two measuring directions run in parallel - for example for the tilted signal paths (16t, 26t) - or are identical - for example for the axial signal paths (16a, 26a). The measurements carried out along the tilted signal paths (16t, 26t) are preferably referred to as quasi-bidirectional, whereas the measurements carried out along the axial signal paths (16a, 26a) are preferably referred to as bidirectional. In figure 6, the positive directions (34, 38) of the (quasi-)bidirectional transmission measurements are indicated with solid lines and solid arrows, whereas the negative directions (36, 40) of the (quasi-)-bidirectional transmission measurements are indicated with dashed lines and/or arrows.

It is noted that in figure 6 only a choice of arrows representing ultrasound based SOS measurements is indicated with regard to the axial signal paths. This is done to provide a better overview. For example, for the left column (28) of the modified sensor arrangement (20), only the arrows from the four emitters (22u, 12u, 12l and 22l) are indicated, which end at the upper left receiver (14u). In the context of the present invention, this preferably means that the four emitters (22u, 12u, 12l and 22l) emit ultrasound signals, which are preferably received and/or detected by the upper left receiver (14u). Preferably, the four emitters (22u, 12u, 12l and 22l) of the left column (28) additionally emit ultrasound signals, which are received by the additional left receiver (24l) and the left lower receiver (14l). This can be seen, when taking into account the arrows plotted with regard to the central column (30) and the right column (32). Preferably, the graphic representation of the central column (30) shows those arrows, i.e. ultrasound signals, which are emitted by the four emitters (22u, 12u, 12l and 22l) and which are received and/or detected by the additional central receiver (24c). It is preferred that the graphic representation of the right column (32) shows those arrows, i.e. ultrasound signals, which are emitted by the four emitters (22u, 12u, 12l and 22l) and which are received and/or detected by the lower receiver (14l). The person skilled in the art will understand from figure 6 that it is preferred in the context of the present invention that the ultrasound signals, which are emitted by the four emitters (22u, 12u, 12l and 22l) of one column (28, 30 or 32) of the modified sensor arrangement (20), are preferably received and/or detected by all three receivers (14u, 24, 14l) of that column (28, 30 or 32). This will get particularly apparent from the overall view of the left part of figure 6 showing the axial signals paths (26a) of the modified sensor arrangement (20).

The right part of figure 6 preferably indicates exemplary courses for the tilted signal paths (26t) of the modified sensor arrangement (20). For the tilted signal paths (26t), all ultrasound signals taking part in the SOS measurements are indicated. It is preferred that the ultrasound signals emitted by the left upper emitter (12u) are received and/or detected by the central upper receiver (14u), by the second additional receiver (24b) and by the right lower receiver (14l). Preferably, the ultrasound signals emitted by the right lower emitter (12l) are received and/or detected by the central lower receiver (14l), by the first additional receiver (24a) and by the left upper receiver (14u). The measurements indicated by these six arrows preferably form the positive tilted signal path (26t+) for the modified sensor arrangement (20).

It is preferred that the ultrasound signals emitted by the right upper emitter (12u) are received and/or detected by the central upper receiver (14u), by the first additional receiver (24a) and by the left lower receiver (12l). Preferably, the ultrasound signals emitted by the left lower emitter (12l) are received and/or detected by the central lower receiver (14l), by the second additional receiver (24b) and by the right upper receiver (14u). The measurements indicated by these six arrows preferably form the negative tilted signal path (26t-) for the modified sensor arrangement (20).

**List of reference signs:**

| | |
|---|---|
| 10 | First sensor arrangement |
| 12 | emitters |
| 12u | upper emitters forming a row of upper emitters of the first sensor arrangement |
| 12l | lower emitters forming a row of lower emitters of the first sensor arrangement |
| 14 | receivers |
| 14u | upper receivers forming a row of upper receivers of the first sensor arrangement |
| 14l | lower receivers forming a row of lower receivers of the first sensor arrangement |
| 16 | signal paths |
| 16a | axial signal paths |
| 16al | left axial signal path |
| 16ac | central axial signal path |
| 16ar | right axial signal path |
| 16t | tilted signal paths |
| 16t+ | positive tilted signal path |
| 16t- | negative tilted signal path |
| 18 | tilting angle *alpha* |
| 18a | potential exemplary position of the tilting angle |

*alpha*

| | |
|---|---|
| 18b | potential exemplary position of the tilting angle |
| 18c | potential exemplary position of the tilting angle |
| 20 | modified sensor arrangement |
| 22 | additional emitters |
| 22u | additional upper emitters forming an uppermost row of emitters of the modified sensor arrangement, first row of additional emitters |
| 22l | additional lower emitters forming a lowest row of emitters of the modified sensor arrangement, second row of additional emitters |
| 24 | additional receivers |
| 24l | left additional receiver |
| 24c | central additional receiver |
| 24r | right additional receiver |
| 24a | first further additional receiver |
| 24b | second further additional receiver |
| 26 | signal paths of the modified sensor arrangement |
| 26a | axial signal paths of the modified sensor arrangement |
| 26al | left axial signal path of the modified sensor arrangement |
| 26ac | central axial signal path of the modified sensor arrangement |
| 26ar | right axial signal path of the modified sensor arrangement |
| 26t | tilted signal paths of the modified sensor arrangement |
| 26t+ | positive tilted signal path of the modified sensor arrangement |
| 26t- | negative tilted signal path of the modified sensor arrangement |
| 28 | left column of sensors |
| 30 | central column of sensor |
| 32 | right column of sensors |
| 34 | positive direction of bidirectional transmission measurement |
| 36 | negative direction of bidirectional transmission measurement |
| 38 | positive direction of quasi-bidirectional transmission measurement |
| 40 | negative direction of quasi-bidirectional transmission measurement |

**Claims**

1. Sensor arrangement (10) for an ultrasonic probe comprising emitters (12) and receivers (14) wherein the emitters (12) and receivers (14) are arranged in rows and columns (28, 30, 32) **characterized in that** the emitters (12) and receivers (14) are arranged in rows of emitters (12) and rows of receivers (14), wherein the columns (28, 30, 32) are formed from upper emitters (12u), upper receivers (14u), lower receivers (14l) and lower emitters (12l), wherein the sensor arrangement (10) comprises electronics configured to excite and receive ultrasonic signals and to measure a velocity of an ultrasonic signal along a number of different signal paths (16) between emitters and receivers, wherein the number of different signal paths (16) comprises axial signal paths (16a) and tilted signal paths (16t), wherein the tilted signal paths (16t) are tilted compared to the axial signal paths (16a) at a tilting +/- angle *alpha* (18), the tilting angle al*pha* (18) being in a range of 30 to 45 °.

2. Sensor arrangement (10) according to claim 1 **characterized in that** a distance between the rows of emitters (12) and rows of receivers (14) is in a range of 5 to 20 mm, preferably in a range of 10 to 15 mm and most preferably at 13 mm.

3. Sensor arrangement (10) according to claim 1 or claim 2 **characterized in that** a distance between the columns (28, 30, 32) of sensors (12, 14) is in a range of 5 to 15 mm, preferably in a range of 8 to 12 mm and most preferably at 10 mm.

4. Sensor arrangement (10) according to one or more of the preceding claims **characterized in that** the sensor arrangement (10) comprises a first column (28), a second column (30) and a third column (32), wherein the first column (28) coincides with a left outer axial signal (16al) path, wherein the second column (30) coincides with a central axial signal path (16ac) and the third column (32) coincides with a right axial signal path (16ar).

5. Sensor arrangement (10) according to one or more of the preceding claims **characterized in that**

   the sensor arrangement (10) comprises six emitters (12) and six receivers (14), wherein three emitters (12) form a row of upper emitters (12u), wherein three receivers (14) form a row of upper receivers (14u), wherein three receivers (14) form a row of lower receivers (14l) and wherein three emitters (12) form a row of lower emitters (12l).

6. Sensor arrangement (10) according to one or more of the preceding claims **characterized in that** the sensor arrangement (10) comprises additional rows of emitters (22u and 22l) and an additional row of receivers (24).

7. Sensor arrangement (10) according to claim 6 **characterized in that** the additional rows of emitters (22) are arranged as a row of additional upper emitters (22u) above the row of upper emitters (12u) and as a row of additional lower emitters (22l) below the row of lower emitters (12l).

8. Sensor arrangement (10) according to claim 6 or claim 7
**characterized in that**
the additional row of receivers (24) is arranged between the existing rows of receivers (14u and 14l).

9. Sensor arrangement (10) according to one or more of claims 6 to 8
**characterized in that**
the additional row of receivers (24) comprises five additional receivers (24l, 24a, 24c, 24b, 24r).

10. Sensor arrangement (10) according to claim 9
**characterized in that**
a distance between the five additional receivers (24l, 24a, 24c, 24b, 24r) is in a range of 1 to 10 mm, preferably 3 to 7 mm, most preferably at 5 mm.

11. Sensor arrangement (10) according to one or more of claims 6 to 10
**characterized in that**
a distance between the additional rows of sensors (22, 24) and the neighboring rows of sensors (12, 14) is in a range of 3 to 15 mm, preferably 4 to 10 mm and most preferred at 6.5 mm.

12. Method for determining the difference in time of flight between a first ultrasonic signal and a second ultrasonic signal,
**comprising the following steps:**

a) Providing a sensor arrangement according to one or more of the preceding claims,
b) Providing a first ultrasonic signal and a second ultrasonic signal,
c) Determining a time of flight (TOF) of the first and the second ultrasonic signal,
d) Determining a difference in time of flight (ΔTOF) between the first and the second ultrasonic signal,
e) Using the ΔTOF in order to determine a speed of sound (SOS).

13. Use of the sensor arrangements (10) according to claims 1 or 11 in order to determine a speed of sound of an ultrasound signal within a bone material.

**Patentansprüche**

1. Sensoranordnung (10) für eine Ultraschallsonde, die Sender (12) und Empfänger (14) umfasst, wobei die Sender (12) und die Empfänger (14) in Reihen und Spalten (28, 30, 32) angeordnet sind,
**dadurch gekennzeichnet, dass**
die Sender (12) und die Empfänger (14) in Reihen von Sendern (12) und Reihen von Empfängern (14) angeordnet sind, wobei die Spalten (28, 30, 32) aus oberen Sendern (12u), oberen Empfängern (14u), unteren Empfängern (14l) und unteren Sendern (12l) gebildet sind, wobei die Sensoranordnung (10) Elektronik umfasst, die dazu konfiguriert ist, Ultraschallsignale anzuregen und zu empfangen und eine Geschwindigkeit eines Ultraschallsignals entlang einer Anzahl unterschiedlicher Signalpfade (16) zwischen Sendern und Empfängern zu messen, wobei die Anzahl unterschiedlicher Signalpfade (16) axiale Signalpfade (16a) und geneigte Signalpfade (16t) umfasst, wobei die geneigten Signalpfade (16t) im Vergleich zu den axialen Signalpfaden (16a) um einen Neigungswinkel +/- *alpha* (18) geneigt sind, wobei der Neigungswinkel *alpha* (18) in einem Bereich von 30 bis 45° liegt.

2. Sensoranordnung (10) nach Anspruch 1,
**dadurch gekennzeichnet, dass**
ein Abstand zwischen den Reihen von Sendern (12) und den Reihen von Empfängern (14) in einem Bereich von 5 bis 20 mm, vorzugsweise in einem Bereich von 10 bis 15 mm und am meisten bevorzugt bei 13 mm liegt.

3. Sensoranordnung (10) nach Anspruch 1 oder Anspruch 2, **dadurch gekennzeichnet, dass**
ein Abstand zwischen den Spalten (28, 30, 32) von Sensoren (12, 14) in einem Bereich von 5 bis 15 mm, vorzugsweise in einem Bereich von 8 bis 12 mm und am meisten bevorzugt bei 10 mm liegt.

4. Sensoranordnung (10) nach einem oder mehreren der vorstehenden Ansprüche,
**dadurch gekennzeichnet, dass**
die Sensoranordnung (10) eine erste Spalte (28), eine zweite Spalte (30) und eine dritte Spalte (32) umfasst, wobei die erste Spalte (28) mit einem linken äußeren axialen Signalpfad (16al) zusammenfällt, wobei die zweite Spalte (30) mit einem mittleren axialen Signalpfad (16ac) zusammenfällt und die dritte Spalte (32) mit einem rechten axialen Signalpfad (16ar) zusammenfällt.

5. Sensoranordnung (10) nach einem oder mehreren der vorstehenden Ansprüche,
**dadurch gekennzeichnet, dass**

die Sensoranordnung (10) sechs Sender (12) und sechs Empfänger (14) umfasst,
wobei drei Sender (12) eine Reihe von oberen Sendern (12u) bilden,
wobei drei Empfänger (14) eine Reihe von oberen Empfängern (14u) bilden,

wobei drei Empfänger (14) eine Reihe von unteren Empfängern (141) bilden und

wobei drei Sender (12) eine Reihe von unteren Sendern (121) bilden.

6. Sensoranordnung (10) nach einem oder mehreren der vorstehenden Ansprüche, **dadurch gekennzeichnet, dass** die Sensoranordnung (10) zusätzliche Reihen von Sendern (22u und 221) und eine zusätzliche Reihe von Empfängern (24) umfasst.

7. Sensoranordnung (10) nach Anspruch 6, **dadurch gekennzeichnet, dass** die zusätzlichen Reihen von Sendern (22) als eine Reihe zusätzlicher oberer Sender (22u) über der Reihe oberer Sender (12u) und als eine Reihe zusätzlicher unterer Sender (221) unter der Reihe unterer Sender (121) angeordnet sind.

8. Sensoranordnung (10) nach Anspruch 6 oder Anspruch 7, **dadurch gekennzeichnet, dass** die zusätzliche Reihe von Empfängern (24) zwischen den bestehenden Reihen von Empfängern (14u und 141) angeordnet ist.

9. Sensoranordnung (10) nach einem oder mehreren der Ansprüche 6 bis 8, **dadurch gekennzeichnet, dass** die zusätzliche Reihe von Empfängern (24) fünf zusätzliche Empfänger (241, 24a, 24c, 24b, 24r) umfasst.

10. Sensoranordnung (10) nach Anspruch 9, **dadurch gekennzeichnet, dass** ein Abstand zwischen den fünf zusätzlichen Empfängern (241, 24a, 24c, 24b, 24r) in einem Bereich von 1 bis 10 mm, vorzugsweise 3 bis 7 mm, am meisten bevorzugt bei 5 mm liegt.

11. Sensoranordnung (10) nach einem oder mehreren der Ansprüche 6 bis 10, **dadurch gekennzeichnet, dass** ein Abstand zwischen den zusätzlichen Reihen von Sensoren (22, 24) und den benachbarten Reihen von Sensoren (12, 14) in einem Bereich von 3 bis 15 mm, vorzugsweise 4 bis 10 mm und am meisten bevorzugt bei 6,5 mm liegt.

12. Verfahren zum Bestimmen der Laufzeitdifferenz zwischen einem ersten Ultraschallsignal und einem zweiten Ultraschallsignal, **umfassend die folgenden Schritte:**

    a) Bereitstellen einer Sensoranordnung nach einem oder mehreren der vorstehenden Ansprüche,
    b) Bereitstellen eines ersten Ultraschallsignals und eines zweiten Ultraschallsignals,
    c) Bestimmen einer Laufzeit (time of flight - TOF) des ersten und des zweiten Ultraschallsignals,
    d) Bestimmen einer Laufzeitdifferenz ($\Delta$TOF) zwischen dem ersten und dem zweiten Ultraschallsignal,
    e) Verwenden der $\Delta$TOF zum Bestimmen einer Schallgeschwindigkeit (speed of sound - SOS).

13. Verwendung der Sensoranordnungen (10) nach Anspruch 1 oder 11, um eine Schallgeschwindigkeit eines Ultraschallsignals innerhalb eines Knochenmaterials zu bestimmen.

**Revendications**

1. Agencement de capteurs (10) pour une sonde à ultrasons comprenant des émetteurs (12) et des récepteurs (14) dans lequel les émetteurs (12) et les récepteurs (14) sont agencés en rangées et colonnes (28, 30, 32) **caractérisé en ce que** les émetteurs (12) et les récepteurs (14) sont agencés en rangées d'émetteurs (12) et en rangées de récepteurs (14), dans lequel les colonnes (28, 30, 32) sont formées d'émetteurs supérieurs (12u), de récepteurs supérieurs (14u), de récepteurs inférieurs (141) et d'émetteurs inférieurs (121), dans lequel l'agencement de capteurs (10) comprend des composants électroniques configurés pour exciter et recevoir des signaux ultrasonores et pour mesurer une vitesse d'un signal ultrasonore le long d'un certain nombre de trajets de signaux différents (16) entre émetteurs et récepteurs, dans lequel le nombre de trajets de signaux différents (16) comprend des trajets de signaux axiaux (16a) et des trajets de signaux inclinés (16t), dans lequel les trajets de signaux inclinés (16t) sont inclinés par rapport aux trajets de signaux axiaux (16a) à un angle d'inclinaison +/*alpha* (18), l'angle d'inclinaison *alpha* (18) étant situé dans une plage allant de 30 à 45°.

2. Agencement de capteurs (10) selon la revendication 1 **caractérisé en ce qu'** une distance entre les rangées d'émetteurs (12) et les rangées de récepteurs (14) est située dans une plage allant de 5 à 20 mm, de préférence dans une plage allant de 10 à 15 mm et étant égale de manière préférée entre toutes à 13 mm.

3. Agencement de capteurs (10) selon la revendication 1 ou la revendication 2 **caractérisé en ce qu'** une distance entre les colonnes (28, 30, 32) de capteurs (12, 14) est située dans une plage allant de 5 à 15 mm, de préférence dans une plage de 8 à 12 mm et étant égale de manière préférée entre toutes à 10 mm.

4. Agencement de capteurs (10) selon une ou plusieurs des revendications précédentes **caractérisé en ce que** l'agencement de capteurs (10) comprend une première colonne (28), une deuxième colonne (30) et une troisième colonne (32), dans lequel la première colonne (28) coïncide avec un trajet de signaux axial extérieur gauche (16al), dans lequel la deuxième colonne (30) coïncide avec un trajet de signaux axial central (16ac) et la troisième colonne (32) coïncide avec un trajet de signaux axial droit (16ar).

5. Agencement de capteurs (10) selon une ou plusieurs des revendications précédentes **caractérisé en ce que**

   l'agencement de capteurs (10) comprend six émetteurs (12) et six récepteurs (14), dans lequel trois émetteurs (12) forment une rangée d'émetteurs supérieurs (12u), dans lequel trois récepteurs (14) forment une rangée de récepteurs supérieurs (14u), dans lequel trois récepteurs (14) forment une rangée de récepteurs inférieurs (14l) et dans lequel trois émetteurs (12) forment une rangée d'émetteurs inférieurs (12l).

6. Agencement de capteurs (10) selon une ou plusieurs des revendications précédentes **caractérisé en ce que** l'agencement de capteurs (10) comprend des rangées supplémentaires d'émetteurs (22u et 22l) et une rangée supplémentaire de récepteurs (24).

7. Agencement de capteurs (10) selon la revendication 6 **caractérisé en ce que** les rangées d'émetteurs supplémentaires (22) sont agencées en une rangée d'émetteurs supérieurs supplémentaires (22u) au-dessus de la rangée d'émetteurs supérieurs (12u) et en une rangée d'émetteurs inférieurs supplémentaires (22l) en dessous de la rangée d'émetteurs inférieurs (12l).

8. Agencement de capteurs (10) selon la revendication 6 ou la revendication 7 **caractérisé en ce que** la rangée supplémentaire de récepteurs (24) est disposée entre les rangées de récepteurs existantes (14u et 14l).

9. Agencement de capteurs (10) selon une ou plusieurs des revendications 6 à 8 **caractérisé en ce que** la rangée supplémentaire de récepteurs (24) comprend cinq récepteurs supplémentaires (24l, 24a, 24c, 24b, 24r).

10. Agencement de capteurs (10) selon la revendication 9 **caractérisé en ce qu'** une distance entre les cinq récepteurs supplémentaires (24l, 24a, 24c, 24b, 24r) est située dans une plage allant de 1 à 10 mm, de préférence de 3 à 7 mm, est égale de manière préférée entre toutes à 5 mm.

11. Agencement de capteurs (10) selon une ou plusieurs des revendications 6 à 10 **caractérisé en ce qu'** une distance entre les rangées supplémentaires de capteurs (22, 24) et les rangées voisines de capteurs (12, 14) est située dans une plage allant de 3 à 15 mm, de préférence de 4 à 10 mm et est égale de manière préférée entre toutes à 6,5 mm.

12. Procédé de détermination de la différence de temps de vol entre un premier signal ultrasonore et un second signal ultrasonore, **comprend les étapes suivantes :**

    a) la fourniture d'un agencement de capteurs selon une ou plusieurs des revendications précédentes,
    b) la fourniture d'un premier signal ultrasonore et d'un second signal ultrasonore,
    c) la détermination d'un temps de vol (TOF) du premier et du second signal ultrasonore,
    d) la détermination d'une différence de temps de vol ($\Delta$TOF) entre le premier et le second signal ultrasonore,
    e) l'utilisation de la $\Delta$TOF pour déterminer une vitesse du son (SOS) .

13. Utilisation des agencements de capteurs (10) selon les revendications 1 ou 11 pour déterminer une vitesse du son d'un signal ultrasonore à l'intérieur d'un matériau osseux.

Fig. 1

Fig. 2

Fig. 3

Fig. 4

Fig. 5

26a

26t

26t-

26t+

26al    26ac    26ar

26

Fig. 6

**EP 3 735 585 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 7112173 B1
- US 2011112404 A1